# FASCICULE DE BREVET EUROPEEN

(11) **EP 4 147 055 B1**
(45) Date de publication et mention de la délivrance du brevet: **13.11.2024**
(21) Numéro de dépôt: 21722487.2
(22) Date de dépôt: 04.05.2021
(51) Int. Cl.: G01N 33/68

(54) **METHODE DE DETECTION DE L'ACTIVATION PLAQUETTAIRE LIEE A L'INFLAMMATION**
VERFAHREN ZUM NACHWEIS VON ENTZÜNDUNGSBEDINGTER BLUTPLÄTTCHENAKTIVIERUNG
METHOD FOR DETECTING INFLAMMATION-RELATED PLATELET ACTIVATION

(30) Priorité: 05.05.2020 FR 2004447
(43) Date de publication de la demande: 15.03.2023
(73) Titulaire: Etablissement Français du Sang, 93210 La Plaine Saint Denis (FR); Université Jean Monnet Saint-Étienne, 42100 Saint-Étienne (FR)
(72) Inventeur: COGNASSE, Fabrice, 42100 SAINT-ETIENNE (FR); NGUYEN-PEYRE, Thi Kim Anh, 77600 BUSSY SAINT GEORGES (FR); GARRAUD, Olivier, 75015 PARIS (FR); HAMZEH-COGNASSE, Hind, 42100 SAINT-ETIENNE (FR)
(74) Mandataire: Lavoix
(86) Numéro de dépôt international: PCT/EP2021/061691
(87) Numéro de publication internationale: WO 2021/224236

(56) Documents cités:
- WO-A1-03/090794
- WO-A1-2004/024906
- WO-A1-2015/065986
- WO-A1-2017/083599
- WO-A1-95/12125
- WO-A1-96/12956
- WO-A2-2007/010240
- US-A1- 2006 019 315

## Description

### Domaine technique

La présente invention concerne la détection de l'activation plaquettaire liée à l'inflammation.

### Etat de la technique

Les plaquettes sanguines sont des cellules anucléées qui jouent un rôle majeur au cours des processus de l'hémostase primaire. Les plaquettes participent également au processus inflammatoire, recouvrant la détection du signal de danger, la libération de facteur solubles et l'interaction avec cellules leucocytaires et endothéliales.

Les plaquettes contiennent dans leurs granules δ et α des facteurs immunomodulateurs de type facteurs de croissance, cytokines (CK), chimiokines (CH) et les motifs moléculaires associés aux dégâts (*DAMP, Damage Associated Molecular Pattern*) qui sont libérés au cours de leur activation. Ces premières observations permettent notamment de faire le lien entre les évènements thrombotiques et inflammatoires observés dans nombreuses pathologies cardiovasculaires (endocardites, athérosclérose). Parmi tous les facteurs libérés, le CD40-Ligand (CD40L) est une molécule fondamentale dans l'activation des leucocytes et cellules endothéliales.

L'implication des plaquettes dans la réponse immunitaire a également été confortée par le fait qu'elles expriment des « Toll like Receptors » (TLR) fonctionnels, récepteurs clés dans la reconnaissance de motifs conservés à la surface des pathogènes. De par cette caractéristique, les plaquettes pourraient alors être considérées comme des sentinelles de l'immunité innée. Les plaquettes interagissent également avec les leucocytes par un contact direct ou indirect via le fibrinogène.

L'ensemble des observations concernant l'implication des plaquettes dans l'immunité innée ont principalement été faites individuellement. Cependant, elles révèlent que les plaquettes possèdent l'arsenal nécessaire pour participer à des mécanismes complexes tels que l'inflammation. D'un point de vue moléculaire, l'inflammation implique des molécules immunomodulatrices, solubles (chimiokines, cytokines) ou cellulaires (sélectines, intégrines), menant à l'extravasation des leucocytes, afin qu'ils puissent exercer leur fonction inflammatoire directement au niveau muqueux.

Ainsi, de nombreux travaux associent des pathologies inflammatoires avec un dysfonctionnement direct ou indirect de la physiologie plaquettaire.

Il serait donc utile de disposer de moyens permettant de détecter facilement l'activation plaquettaire liée à l'inflammation.

WO2017/083599 décrit une méthode de détection de l'activation plaquettaire associée à la pathogénèse de maladies vasculaires, en utilisant des biomarqueurs d'exosomes dérivés des plaquettes, dont CD63, CD62P ou CD40.

WO2007/010240 décrit l'utilisation d'anticorps anti-CD63, ou anti-CD62P pour la capture des plaquettes activées.

WO95/12125 et WO96/12956 décrivent une méthode pour mesurer l'activité plaquettaire dans un échantillon de plaquettes, comprenant l'utilisation d'un activateur des plaquettes et d'un anticorps anti-sélectine P (CD62P).

WO2015/065986 et WO2004/024906 décrivent que l'activation plaquettaire peut être déterminée, entre autres, en mesurant une augmentation de l'expression de la sélectine P.

US2006/019315 décrit une méthode de diagnostic ou de détermination d'un risque d'une complication cardiovasculaire pouvant inclure la mesure d'un marqueur de l'activation plaquettaire, notamment sCD40L, vWF et des variants de ceux-ci.

WO03/090794 décrit que RANTES est connu pour être libéré des plaquettes activées.

### Description détaillée

De manière surprenante, les Inventeurs ont mis en évidence un panel de sept biomarqueurs nécessaires et suffisants pour permettre de détecter efficacement une activation plaquettaire liée à l'inflammation.

Ce panel de biomarqueurs a été identifié parmi 47 molécules intra-plaquettaires, solubles ou membranaires, préalablement sélectionnées par les Inventeurs parmi un ensemble beaucoup plus large de molécules potentiellement associées à l'activation plaquettaire.

A cet effet, des conditions expérimentales *in vitro* se rapprochant de celles retrouvées dans la circulation sanguine ont été utilisées. Des plasmas riches en plaquettes (PRP) issus de 10 donneurs ont ainsi été soumis ou non à six conditions de stimulation différentes liée à l'inflammation: un agoniste de PAR-1, un agoniste de PAR-4, l'ADP (Adénosine Diphosphate), le collagène, sCD40L (ligand soluble de CD40) ou le fibrinogène.

Une analyse biomathématique des données obtenues pour ces 47 paramètres biologiques dans les 6 conditions de stimulation ou non a ainsi conduit à l'identification des sept molécules suivantes, qualifiées de biomarqueurs, permettant à elles seules de détecter une activation plaquettaire liée à l'inflammation : AKT, PKC, CD62P, CD63, RANTES, TSLP et CD40 ligand.

Cette analyse biomathématique a également permis d'identifier des biomarqueurs spécifiques de l'activation d'une voie de signalisation intra-plaquettaire donnée.

La mise en évidence de ce panel de sept biomarqueurs permet ainsi pour la première fois de détecter spécifiquement l'activation plaquettaire liée à l'inflammation.

Ce panel de sept biomarqueurs permet avantageusement de limiter le nombre de molécules testées pour caractériser l'activation plaquettaire.

La mesure de la quantité, concentration et/ou proportion de ces sept biomarqueurs dans un contexte physiopathologique permet ainsi avantageusement de détecter une activation plaquettaire liée à l'inflammation.

Un premier objet de l'invention est une méthode de détection d'une activation plaquettaire liée à l'inflammation, comprenant de mesurer la quantité, concentration et/ou proportion des biomarqueurs AKT, PKC, CD62P, CD63, RANTES, TSLP et CD40 ligand dans un échantillon biologique comprenant des plaquettes.

Un autre objet de l'invention est une méthode de diagnostic d'une activation plaquettaire liée à l'inflammation chez un sujet, comprenant :
a) mesurer la quantité, concentration et/ou proportion des biomarqueurs AKT, PKC, CD62P, CD63, RANTES, TSLP et CD40 ligand dans un échantillon biologique comprenant des plaquettes dudit sujet,
b) comparer les résultats obtenus à l'étape a) avec une valeur contrôle standard correspondante, et
c) en déduire si le sujet présente une activation plaquettaire liée à l'inflammation.

L'étape c) de la méthode de diagnostic d'une activation plaquettaire liée à l'inflammation telle que définie ci-dessus peut comprendre d'en déduire si une voie de signalisation intra-plaquettaire spécifique est activée.

Un autre objet de l'invention est une méthode de suivi de l'efficacité d'un traitement curatif ou préventif d'une maladie inflammatoire chez un sujet, comprenant :
a) mesurer la quantité, concentration et/ou proportion des biomarqueurs AKT, PKC, CD62P, CD63, RANTES, TSLP et CD40 ligand dans un échantillon biologique comprenant des plaquettes dudit sujet à un instant t au cours dudit traitement,
b) comparer les résultats obtenus à l'étape a) avec une valeur contrôle standard correspondante et/ou avec une valeur correspondante obtenue avant le début dudit traitement ou à un instant au cours du traitement qui est antérieur à l'instant t,
c) en déduire si le traitement est efficace, et
d) optionnellement, répéter les étapes a) à c).

Un autre objet de l'invention est une méthode de suivi de l'évolution d'une maladie inflammatoire associée à une activation plaquettaire liée à l'inflammation chez un sujet, comprenant :
a) mesurer la quantité, concentration et/ou proportion des biomarqueurs AKT, PKC, CD62P, CD63, RANTES, TSLP et CD40 ligand dans un échantillon biologique comprenant des plaquettes dudit sujet à deux instants t1 et t2 séparés dans le temps,
b) comparer les résultats à l'instant t1 et à l'instant t2 obtenus à l'étape a),
c) en déduire si la maladie inflammatoire évolue favorablement, et
d) optionnellement, répéter les étapes a) à c).

Un autre objet de l'invention est une méthode de stratification d'un sujet souffrant ou à risque de souffrir d'une maladie inflammatoire dans une catégorie de sujets avec activation plaquettaire liée à l'inflammation ou dans une catégorie de sujets sans activation plaquettaire liée à l'inflammation, comprenant :
a) mesurer la quantité, concentration et/ou proportion des biomarqueurs AKT, PKC, CD62P, CD63, RANTES, TSLP et CD40 ligand dans un échantillon biologique comprenant des plaquettes dudit sujet,
b) comparer les résultats obtenus à l'étape a) avec une valeur contrôle standard correspondante, et
c) en déduire si le sujet est dans la catégorie de sujets avec activation plaquettaire liée à l'inflammation ou dans la catégorie de sujets sans activation plaquettaire liée à l'inflammation.

Dans les méthodes telle que définies ci-dessus, la maladie inflammatoire est par exemple sélectionnée dans le groupe consistant en athérosclérose, inflammation pulmonaire, polyarthrite rhumatoïde (PR), maladie inflammatoire chronique de l'intestin (MICI), sepsis, sepsis sévère, choc septique, cancer et leurs combinaisons.

Dans les méthodes telle que définies ci-dessus, l'échantillon biologique est de préférence un échantillon de plasma riche en plaquettes.

Un autre objet de l'invention concerne l'utilisation d'un kit comprenant des moyens de détection d'au moins sept biomarqueurs, caractérisé en ce que lesdits au moins sept biomarqueurs sont AKT, PKC, CD62P, CD63, RANTES, TSLP et CD40 ligand pour la détection d'une activation plaquettaire liée à l'inflammation.

Un autre objet de l'invention concerne l'utilisation d'un panel de biomarqueurs comprenant AKT, PKC, CD62P, CD63, RANTES, TSLP et CD40 ligand pour la détection d'un activation plaquettaire liée à l'inflammation.

### Echantillon biologique

La détection d'une activation plaquettaire liée à l'inflammation est évaluée dans un échantillon biologique.

L'échantillon biologique est un échantillon comprenant des plaquettes.

Par « plaquettes », on désigne ici les plaquettes sanguines et les plaquettes de culture.

Par « plaquettes sanguines », appelées également « thrombocytes », on désigne ici des cellules sans noyau circulant dans le sang. Les plaquettes sanguines sont formées dans la moelle osseuse, à partir du fractionnement de mégacaryocytes.

Par « plaquettes de culture », on désigne ici des plaquettes obtenues à partir de mégacaryocytes cultivés *in vitro.* Le mégacaryocyte provient alors de préférence d'une lignée de mégacaryocytes immortalisés.

L'échantillon biologique peut comprendre des plaquettes sanguines ou des plaquettes de culture.

L'échantillon biologique comprenant des plaquettes sanguines peut être un échantillon provenant d'un ou plusieurs sujets.

L'échantillon biologique comprenant des plaquettes de culture peut être un échantillon de milieu de culture.

L'échantillon biologique est de préférence un échantillon provenant d'un ou plusieurs sujets.

Le ou les sujets sont par exemple tel que définis ci-dessous dans la section « Sujet ».

Un échantillon biologique préféré selon l'invention est un échantillon de plasma riche en plaquettes (PRP).

L'échantillon de plasma riche en plaquettes peut être obtenu par toute méthode approprié bien connue de l'homme du métier.

Par exemple, un échantillon de plasma riche en plaquettes peut être obtenu par centrifugation d'un échantillon de sang, par exemple à 192 g pendant 5 à 10 minutes, et récupération de la phase supérieure correspondant au plasma riche en plaquettes.

L'échantillon de plasma riche en plaquettes peut provenir d'un sujet, être un mélange de plasmas riches en plaquettes provenant d'au moins deux sujets ou un plasma riche en plaquettes obtenu à partir d'un mélange de sang provenant d'au moins deux sujets.

L'échantillon de plasma riche en plaquettes provient de préférence d'un sujet.

### Sujet

Le sujet est de préférence un mammifère, par exemple un sujet humain ou un mammifère non humain, tel qu'un chat, chien, singe, lapin, souris ou rat.

Le sujet humain peut être un homme ou une femme de tout âge, tel qu'un nourrisson, un enfant, un adolescent, un adulte ou une personne âgée.

Le sujet est de préférence un sujet souffrant ou à risque de souffrir d'une maladie inflammatoire.

Un sujet à risque de souffrir d'une maladie inflammatoire est par exemple un sujet présentant des antécédents familiaux de maladie inflammatoire, ayant déjà souffert d'au moins une maladie inflammatoire, présentant des facteurs de risques génétiques, métaboliques et/ou liés au mode de vie et/ou présentant au moins un symptôme pouvant être précurseur d'une maladie inflammatoire.

La maladie inflammatoire est notamment telle que définie ci-dessous dans la section « maladie inflammatoire ».

### Maladie inflammatoire

La maladie inflammatoire, appelée également pathologie inflammatoire, peut être toute maladie résultant d'une réaction anormale du système immunitaire.

La maladie inflammatoire peut être une maladie auto-inflammatoire, une maladie auto-immune ou une affection inflammatoire d'origine indéterminée.

Les pathologies inflammatoires se définissent par une agression tissulaire en raison d'un dérèglement du système immunitaire. Aussi, parmi ces dernières, on peut citer l'athérosclérose, polyarthrite rhumatoïde, inflammation pulmonaire, maladie inflammatoire chronique de l'intestin, sepsis, sepsis sévère, choc septique ou cancer.

La maladie inflammatoire est ainsi de préférence sélectionnée dans le groupe consistant en l'athérosclérose, polyarthrite rhumatoïde, inflammation pulmonaire, maladie inflammatoire chronique de l'intestin, sepsis, sepsis sévère, choc septique, cancer et leurs combinaisons.

L'athérosclérose (appelée également artériosclérose) est une maladie touchant les artères et caractérisée par l'apparition de plaques d'athérome sur la paroi interne des artères.

La polyarthrite rhumatoïde (ou PR) est une maladie systémique du tissu conjonctif caractérisée par une inflammation articulaire chronique évoluant par poussées.

L'inflammation pulmonaire comprend notamment la maladie pulmonaire inflammatoire et/ou l'allergie pulmonaire.

La maladie pulmonaire inflammatoire comprend notamment l'atteinte pulmonaire aiguë (« *Acute Lung Ingury* » ou ALI, également appelé « ARDS modéré »), le syndrome de détresse respiratoire aiguë (« *Acute Respiratory Distress Syndrome ou ARDS*)*,* l'atteinte pulmonaire aiguë résultant d'une transfusion (« *Transfusion Related Acute Lung Injury* » ou TRALI).

L'allergie pulmonaire, appelée également allergie respiratoire, est une maladie alternant une phases aiguë (appelée également crise d'asthme) et une phase chronique.

La maladie inflammatoire chronique de l'intestin (ou MICI) peut être sélectionnée parmi la maladie de Crohn et la rectocolite hémorragique.

Une réaction inflammatoire excessive, en réponse à une infection, provoque le sepsis et ses formes les plus graves, le sepsis sévère et le choc septique.

La cellule cancéreuse va provoquer une inflammation et envahir les cellules voisines pour créer un climat propice à la croissance de cellules de proximité. C'est ainsi que, petit à petit, naît la tumeur cancéreuse. Concrètement, la cellule cancéreuse utilise l'inflammation pour progresser.

### Biomaraueurs

La présente invention a notamment pour objet un panel de sept biomarqueurs utile pour détecter une activation plaquettaire liée à l'inflammation. Ces sept biomarqueurs sont CD62P, CD63, AKT, PKC, RANTES, TSLP et CD40 ligand.

CD62P et CD63 sont des protéines membranaires.

CD62P (appelée également sélectine P, GMP-140 ou PADGEM) est une glycoprotéine codée par le gène SELP chez l'homme. Elle est exprimée à la surface des plaquettes sanguines et les cellules endothéliales activées. CD62P fonctionne comme une molécule d'adhésion cellulaire à la surface des cellules endothéliales activées et des plaquettes activées.

CD63 est une protéine codée par le gène CD63 chez l'homme.

AKT et PKC sont des protéines intracellulaires.

AKT (appelée également AKT1 ou « protéine kinase B ») est une protéine sérine-thréonine kinase codée par le gène Akt1 chez l'homme.

PKC (appelée également « protéine kinase C ») est une protéine sérine-thréonine kinase.

RANTES, TSLP et CD40 ligand sont des protéines solubles.

RANTES (appelée également CCL5) est une protéine codée par le gène CCL5 chez l'homme.

TSLP (appelée également « lymphopoïétine stromale thymique ») est une protéine codée par le gène TSLP chez l'homme.

CD40 ligand (appelée également CD40L ou CD154) est une glycoprotéine transmembranaire membre de la superfamille du TNF (*Tumor Necrosis Factor*) qui présente une forme membranaire après activation, puis une forme soluble après clivage.

### Méthode de détection d'une activation plaquettaire liée à l'inflammation

Disposer d'une méthode permettant de détecter une activation plaquettaire liée à l'inflammation peut s'avérer particulièrement utile dans de nombreuses applications, telles que dans la recherche et développement et/ou dans le domaine médical.

Par exemple, en recherche et développement, la détection d'une activation plaquettaire liée à l'inflammation peut permettre de tester l'activation plaquettaire liée à l'inflammation, par exemple pour tester la capacité d'une molécule à induire ou inhibant l'activation plaquettaire liée à l'inflammation et/ou de développer un modèle d'activation plaquettaire liée à l'inflammation ou permettant de tester l'activation plaquettaire liée à l'inflammation.

Par exemple, dans le domaine médical, la détection d'une activation plaquettaire liée à l'inflammation peut permettre d'établir un diagnostic ou d'aider au diagnostic d'un sujet souffrant ou à risque de souffrir d'une maladie inflammatoire, de stratifier un sujet souffrant ou susceptible de souffrir d'une maladie inflammatoire dans la catégorie « sujet présentant une activation plaquettaire liée à l'inflammation » ou dans la catégorie « sujet sans activation plaquettaire liée à l'inflammation », de suivre l'évolution d'une maladie inflammatoire au cours du temps, et/ou suivre l'efficacité d'un traitement curatif ou préventif d'une maladie inflammatoire.

La présente invention a ainsi particulièrement pour objet une méthode, en particulier une méthode *in vitro,* de détection d'une activation plaquettaire liée à l'inflammation, comprenant de mesurer la quantité, concentration et/ou proportion des biomarqueurs AKT, PKC, CD62P, CD63, RANTES, TSLP et CD40 ligand dans un échantillon biologique comprenant des plaquettes.

Par « proportion d'un biomarqueur », on désigne par exemple le pourcentage de cellules exprimant ledit biomarqueur dans une population cellulaire, en particulier dans une population de plaquettes. La mesure de la proportion d'un biomarqueur est alors une mesure de l'expression dudit biomarqueur.

L'échantillon biologique est notamment tel que défini ci-dessus.

La méthode telle que définie ci-dessus peut notamment comprendre :
a) mesurer la quantité, concentration et/ou proportion des biomarqueurs AKT, PKC, CD62P, CD63, RANTES, TSLP et CD40 ligand dans un échantillon biologique comprenant des plaquettes,
b) optionnellement, comparer les résultats obtenus à l'étape a) avec une valeur contrôle standard correspondante, et
c) optionnellement, en déduire si une activation plaquettaire est présente dans l'échantillon biologique.

### Etape a)

Dans l'étape a), la quantité, concentration et/ou proportion des biomarqueurs AKT, PKC, CD62P, CD63, RANTES, TSLP et CD40 ligand est mesurée dans un échantillon biologique comprenant des plaquettes.

La mesure de la quantité, concentration et/ou proportion d'un biomarqueur dans un échantillon biologique peut être effectuée directement dans l'échantillon biologique ou après traitement de l'échantillon biologique.

Par « traitement de l'échantillon biologique », on désigne ici au moins une étape de traitement, par exemple sélectionnée dans le groupe consistant en une centrifugation, un cycle de congélation puis décongélation, une lyse, une mise en suspension dans un tampon et un lavage.

Selon le biomarqueur, la mesure de la quantité, concentration et/ou proportion de ce biomarqueur peut ainsi être mesurée directement dans l'échantillon biologique ou après traitement de l'échantillon biologique, par exemple dans un surnageant des plaquettes, dans un lysat plaquettaire, dans un surnageant de lysat plaquettaire et/ou dans un culot de plaquettes, ledit culot de plaquettes étant de préférence remis en suspension dans un tampon.

Par « surnageant des plaquettes » (appelé également « surnageant plaquettaire »), on désigne ici le surnageant obtenu après centrifugation de l'échantillon biologique, par exemple à 490 g pendant 10 minutes.

Par « culot de plaquettes », on désigne ici le culot obtenu après centrifugation de l'échantillon biologique, par exemple à 490 g pendant 10 minutes.

Le culot de plaquettes peut être remis en suspension dans un tampon, tel que du « PBS » (Phosphate-Buffered Saline) sans calcium sans magnésium ou un tampon de type « Tyrode ».

Par « lysat plaquettaire », on désigne ici le lysat obtenu après lyse des plaquettes.

Le lysat plaquettaire peut par exemple être obtenu en centrifugeant l'échantillon biologique pour obtenir un culot de plaquettes, puis en mettant en suspension au moins une partie dudit culot de plaquettes dans un tampon de lyse. Alternativement, le lysat plaquettaire peut être obtenu par congélation, puis décongélation des plaquettes, par exemple par congélation, puis décongélation de l'échantillon biologique, d'au moins une partie du culot de plaquettes et/ou d'au moins une partie dudit culot de plaquettes remis en suspension dans un tampon, tel que du « PBS » (Phosphate-Buffered Saline) sans calcium sans magnésium ou un tampon de type « Tyrode ».

Par « surnageant du lysat plaquettaire », on désigne ici le surnageant obtenu après centrifugation du lysat plaquettaire, par exemple à 490 g pendant 10 min.

### (i) CD62P et CD63

La mesure de la quantité, concentration et/ou proportion des biomarqueurs CD62P et CD63 peut être effectuée par tout méthode bien connue de l'homme du métier pour mesurer la quantité, concentration et/ou proportion d'une protéine membranaire.

Par exemple, la quantité, concentration et/ou proportion des biomarqueurs CD62P et CD63 est mesurée par cytométrie en flux ou des techniques apparentées de comptage et de mesure des propriétés des cellules (telles que taille, contenu cellulaire, fluorescence, ...).

L'étape a) telle que définie ci-dessus comprend par exemple de mesurer la proportion du biomarqueur CD62P et/ou du biomarqueur CD63 en pourcentage et/ou de mesurer la quantité du biomarqueur CD62P et/ou du biomarqueur CD63 en intensité moyenne de fluorescence (appelée également MFI (*Mean Fluorescence Intensity*))*.*

L'intensité moyenne de fluorescence est de préférence mesurée par cytométrie en flux dans la fenêtre correspondant aux plaquettes. La fenêtre correspondant aux plaquettes est par exemple définie en utilisant le marqueur CD41 qui est caractéristique des plaquettes.

La proportion du biomarqueur, en particulier CD62P ou CD63, correspond au pourcentage de cellules exprimant ledit biomarqueur parmi les cellules comprises dans la fenêtre correspondant aux plaquettes. La proportion du biomarqueur est de préférence obtenue par cytométrie de flux.

L'étape a) telle que définie ci-dessus comprend de préférence de mesurer la proportion des biomarqueurs CD62P et CD63 en pourcentage.

La quantité, concentration et/ou proportion du biomarqueur CD62P et/ou CD63 peut être mesurée directement dans l'échantillon biologique ou dans une partie du culot de plaquettes, de préférence remis en suspension dans un tampon, tel que du « PBS » (Phosphate-Buffered Saline) sans calcium sans magnésium ou un tampon de type « Tyrode ».

### (ii) AKT et PKC

La mesure de la quantité, concentration et/ou proportion des biomarqueurs AKT et PKC peut être effectuée par tout méthode bien connue de l'homme du métier pour mesurer la quantité, concentration et/ou proportion d'une protéine intracellulaire.

Par exemple, leur quantité, concentration et/ou proportion est mesurée dans le lysat plaquettaire, dans le surnageant du lysat plaquettaire et/ou dans le surnageant des plaquettes.

### (iii) RANTES, TSLP et CD40 ligand

La mesure de la quantité, concentration et/ou proportion des biomarqueurs RANTES, TSLP et CD40 ligand peut être effectuée par tout méthode bien connue de l'homme du métier pour mesurer la quantité, concentration et/ou proportion d'une protéine soluble.

Par exemple, leur quantité, concentration et/ou proportion est mesurée dans le surnageant des plaquettes.

Tout type de test permettant de détecter et/ou quantifier spécifiquement le biomarqueur présent dans un milieu liquide (par exemple l'échantillon biologique, le culot de plaquettes remis en suspension dans un tampon, le lysat plaquettaire, le surnageant du lysat plaquettaire ou le surnageant des plaquettes) peut être utilisé, tel qu'un test immunologique (par exemple un test immunologique multiplexe, de préférence mis en oeuvre sur spots ou sur billes), ou Western blot.

De préférence, l'étape a) telle que définie ci-dessus comprend la mesure de la concentration en AKT, PKC, RANTES, TSLP et CD40 ligand, par exemple en masse par le nombre plaquettes (notamment après avoir fait un comptage plaquettaire, par exemple de type NFS (*Numération et Formule Sanguine*))*.* Par exemple, la concentration est exprimée en ng/10⁹ plaquette.

Ainsi, l'étape a) telle que définie ci-dessus peut comprendre :
- centrifuger au moins une partie de l'échantillon biologique comprenant des plaquettes pour obtenir un culot de plaquettes et un surnageant des plaquettes,
- optionnellement, lyser une partie du culot de plaquettes, pour obtenir un lysat plaquettaire, par exemple au moyen d'un tampon de lyse ou par congélation puis décongélation, et, optionnellement, centrifuger ledit lysat plaquettaire, pour obtenir un surnageant du lysat plaquettaire,
- mesurer la quantité, concentration et/ou proportion :
   - des biomarqueurs AKT et PKC dans ledit surnageant des plaquettes, ledit lysat plaquettaire et/ou dans ledit surnageant du lysat plaquettaire,
   - des biomarqueurs RANTES, TSLP et CD40 ligand dans ledit surnageant des plaquettes, et
   - des biomarqueurs CD62P et CD63 dans une partie de l'échantillon biologique et/ou dans une partie dudit culot de plaquettes, optionnellement remis en suspension dans un tampon.

### Etape b)

Dans l'étape b), les résultats obtenus à l'étape a) sont comparés avec une valeur contrôle standard correspondante.

Les résultats obtenus à l'étape a) sont la quantité, concentration et/ou proportion des biomarqueurs AKT, PKC, CD62P, CD63, RANTES, TSLP et CD40 ligand dans l'échantillon biologique.

Le terme « correspondante » dans l'expression « valeur contrôle standard correspondante » signifie que, pour chaque biomarqueur, sa quantité, concentration et/ou proportion mesurée dans l'échantillon biologique est comparée à une valeur contrôle standard de ce biomarqueur.

Si c'est la quantité du biomarqueur qui est mesurée dans l'échantillon biologique, la valeur contrôle standard est une quantité.

Si c'est la concentration du biomarqueur qui est mesurée dans l'échantillon biologique, la valeur contrôle standard est une concentration.

Si c'est la proportion du biomarqueur qui est mesurée dans l'échantillon biologique, la valeur contrôle standard est une proportion.

Une valeur de contrôle standard, appelée également valeur de référence, d'un biomarqueur X peut par exemple être la moyenne, la valeur seuil maximale et/ou la valeur seuil minimale de la quantité, concentration ou proportion de ce biomarqueur X mesurée dans les échantillons provenant d'une population de référence, de préférence constituée de sujets sains ou de sujets souffrant d'une maladie inflammatoire avant traitement.

Les valeurs mesurées dans la population de référence sont effectuées en utilisant une méthode de mesure similaire à celle utilisée pour l'échantillon biologique.

Deux méthodes de mesure sont similaires si, mises en oeuvre sur un même échantillon, elles donnent un résultat identique ou similaire. A cet égard, le calcul du coefficient de variation (CV) est une mesure importante de la fiabilité de la méthode qui est acceptable si CV ≤ 20%.

La valeur seuil minimale et la valeur seuil maximale définissent un intervalle de référence. L'intervalle de référence est généralement défini de manière à comprendre 95% des valeurs obtenues dans la population de référence.

Par « population de référence », on désigne une population d'individus dans laquelle le biomarqueur X est mesuré.

La population de référence peut comprendre au moins 5 sujets, par exemple 10 sujets ou au moins 10 sujets, par exemple au moins 50 sujets ou au moins 100 sujets.

La population de référence est de préférence constituée d'individus sains, c'est-à-dire présentant un bon état de santé général.

La valeur contrôle standard d'un biomarqueur X peut être la moyenne, la valeur seuil maximale et/ou la valeur seuil minimale de la quantité, concentration ou proportion de ce biomarqueur X mesurée dans les échantillons provenant d'une population de référence constituée d'individus sains, les échantillons ayant été ou non stimulées avec un stimulateur plaquettaire.

Le stimulateur plaquettaire est par exemple sélectionné dans le groupe consistant en un agoniste de PAR-1 (par exemple TRAP de séquence SEQ ID NO: 1), un agoniste du PAR-4 (par exemple le peptide de séquence SEQ ID NO: 2), l'ADP, le collagène, sCD40L (ligand soluble de CD40) ou le fibrinogène.

Une valeur contrôle standard peut par exemple correspondre à la moyenne des valeurs obtenues dans les échantillons provenant de 10 sujets sains, sans stimulation des plaquettes.

Une valeur contrôle standard peut ainsi également correspondre à la moyenne des valeurs obtenues dans les échantillons provenant de 10 sujets sains, avec stimulation des plaquettes, en particulier en utilisant un stimulateur plaquettaire sélectionné dans le groupe consistant en un agoniste de PAR-1 (par exemple TRAP de séquence SEQ ID NO: 1), un agoniste du PAR-4 (par exemple le peptide de séquence SEQ ID NO: 2), l'ADP, le collagène, sCD40L (ligand soluble de CD40) ou le fibrinogène.

La population de référence peut alternativement être constituée d'individus présentant une activation plaquettaire liée à l'inflammation, de préférence souffrant d'une même maladie inflammatoire.

Alternativement, la valeur contrôle standard peut être une valeur fixée, par exemple par une administration, cette valeur ne correspondant pas nécessairement à la moyenne, valeur seuil minimale ou valeur seuil maximale des valeurs d'un biomarqueur donné dans une population de référence.

Une valeur contrôle standard correspondant à une population de sujets sains (en particulier une valeur fixée ou une valeur obtenue à partir d'échantillons non soumis à une activation plaquettaire et provenant d'une population de référence constituée d'individus sains) est qualifiée de valeur contrôle standard NA (non activée).

Une valeur contrôle standard correspondant à une population de sujets présentant une activation plaquettaire liée à l'inflammation (en particulier une valeur fixée, une valeur obtenue à partir d'échantillons soumis à une activation plaquettaire et provenant d'une population de référence constituée d'individus sains ou une valeur obtenue à partir d'échantillons provenant d'une population de référence constituée d'individus présentant une activation plaquettaire liée à l'inflammation) est qualifiée de valeur contrôle standard A (activée).

Une valeur contrôle standard A est nécessairement différente d'une valeur standard NA.

S'agissant des marqueurs AKT, PKC, CD62P, CD63, RANTES, TSLP et CD40 ligand, la valeur contrôle standard A est nécessairement supérieure à la valeur standard NA.

Si la quantité, concentration et/ou proportion du biomarqueur AKT, PKC, CD62P, CD63, RANTES, TSLP ou CD40 ligand est :
- supérieure ou égale à une valeur contrôle standard A (en particulier une valeur seuil minimale), et/ou
- supérieure à une valeur contrôle standard NA (en particulier à une valeur seuil maximale),
le biomarqueur est qualifié d'activé.

Si la quantité, concentration et/ou proportion du biomarqueur AKT, PKC, CD62P, CD63, RANTES, TSLP ou CD40 ligand est :
- inférieure à une valeur contrôle standard A (en particulier une valeur seuil minimale), et/ou
- inférieure ou égale à une valeur contrôle standard NA (en particulier à une valeur seuil maximale),
le biomarqueur est qualifié de non activé.

### Etape c)

Dans l'étape c), il est déduit la présence ou non d'une activation plaquettaire liée à l'inflammation dans l'échantillon biologique comprenant des plaquettes.

En particulier, si la quantité, concentration et/ou proportion d'au moins trois desdits biomarqueurs est supérieure à une valeur contrôle standard NA correspondante (en particulier à la valeur seuil maximale ou à une valeur fixée), il en est déduit la présence d'une activation plaquettaire liée à l'inflammation dans l'échantillon biologique.

En particulier, si la quantité, concentration et/ou proportion d'au moins cinq desdits biomarqueurs est inférieure ou égale à la valeur contrôle standard NA correspondante (en particulier à la valeur seuil maximale ou à une valeur fixée), il en est déduit l'absence d'activation plaquettaire liée à l'inflammation dans l'échantillon biologique.

En particulier, si la quantité, concentration et/ou proportion d'au moins trois desdits biomarqueurs est supérieure ou égale à une valeur contrôle standard A correspondante (en particulier à la valeur seuil minimale ou à une valeur fixée), il en est déduit la présence d'une activation plaquettaire liée à l'inflammation dans l'échantillon biologique.

En particulier, si la quantité, concentration et/ou proportion d'au moins cinq desdits biomarqueurs est inférieure à la valeur contrôle standard A correspondante (en particulier à la valeur seuil minimale ou à une valeur fixée), il en est déduit l'absence d'activation plaquettaire liée à l'inflammation dans l'échantillon biologique.

### Méthode de diagnostic d'une activation plaquettaire liée à l'inflammation

La présente invention a également pour objet une méthode de diagnostic d'une activation plaquettaire liée à l'inflammation chez un sujet, comprenant :
a) mesurer la quantité, concentration et/ou proportion des biomarqueurs AKT, PKC, CD62P, CD63, RANTES, TSLP et CD40 ligand dans un échantillon biologique comprenant des plaquettes dudit sujet,
b) comparer les résultats obtenus à l'étape a) avec une valeur contrôle standard correspondante, et
c) en déduire si le sujet présente une activation plaquettaire liée à l'inflammation.

L'échantillon biologique est un échantillon provenant d'un sujet, notamment tel que défini ci-dessus dans la section « Echantillon ».

Le sujet est notamment tel que défini ci-dessus dans la section « sujet ».

Le sujet est de préférence un sujet souffrant ou à risque de souffrir d'une maladie inflammatoire, en particulier une maladie inflammatoire telle que définie ci-dessus dans la section « maladie inflammatoire ».

Les étapes a) et b) sont notamment telles définies ci-dessus dans la section « méthode de détection d'une inflammation plaquettaire ».

Dans l'étape c), il en est déduit si le sujet présente une activation plaquettaire liée à l'inflammation.

En particulier, si la quantité, concentration et/ou proportion d'au moins trois desdits biomarqueurs est supérieure à une valeur contrôle standard NA correspondante (en particulier à la valeur seuil maximale ou à une valeur fixée), il en est déduit que le sujet présente une activation plaquettaire liée à l'inflammation.

En particulier, si la quantité, concentration et/ou proportion d'au moins cinq desdits biomarqueurs est inférieure ou égale à la valeur contrôle standard NA correspondante (en particulier à la valeur seuil maximale ou à une valeur fixée), il en est déduit que le sujet ne présente pas une activation plaquettaire liée à l'inflammation.

En particulier, si la quantité, concentration et/ou proportion d'au moins trois desdits biomarqueurs est supérieure ou égale à une valeur contrôle standard A correspondante (en particulier à la valeur seuil minimale ou à une valeur fixée), il en est déduit que le sujet présente une activation plaquettaire liée à l'inflammation.

En particulier, si la quantité, concentration et/ou proportion d'au moins cinq desdits biomarqueurs est inférieure à la valeur contrôle standard A correspondante (en particulier à la valeur seuil minimale ou à une valeur fixée), il en est déduit que le sujet ne présente pas une activation plaquettaire liée à l'inflammation.

L'étape c) peut comprendre d'en déduire si une voie de signalisation intra-plaquettaire spécifique est activée.

Par exemple,
- si les sept biomarqueurs AKT, PKC, CD62P, CD63, RANTES, TSLP et CD40 ligand sont activés, la voie de signalisation PAR-4 est activée,
- si les biomarqueurs AKT, CD62P, CD63, RANTES et CD40 sont activés, mais pas les biomarqueurs PKC et TSLP, la voie de signalisation PAR-1 est activée,
- si les biomarqueurs AKT, CD40L et TSLP sont activés, mais pas les biomarqueurs PKC, CD62P, CD63 et RANTES, la voie de signalisation du fibrinogène est activée,
- si le biomarqueur CD40L n'est pas activé, mais qu'au moins trois biomarqueurs sont activés, la voie de signalisation sCD40L est activée,
- si les biomarqueurs AKT, PKC, CD62P, RANTES et CD40 ligand sont activés, mais pas les biomarqueurs TSLP et CD63, la voie de signalisation ADP est activée, ou
- si les biomarqueurs AKT, CD62P, RANTES, TSLP et CD40 ligand sont activés, mais pas PKC, la voie de signalisation collagène est activée.

### Méthode de prévention et/ou traitement d'une maladie inflammatoire associée à une activation plaquettaire liée à l'inflammation

La présente invention a également pour objet une méthode de prévention et/ou traitement d'un maladie inflammatoire associée à une activation plaquettaire liée à l'inflammation chez un sujet comprenant :
a) mesurer la quantité, concentration et/ou proportion des biomarqueurs AKT, PKC, CD62P, CD63, RANTES, TSLP et CD40 ligand dans un échantillon biologique comprenant des plaquettes dudit sujet,
b) comparer les résultats obtenus à l'étape a) avec une valeur contrôle standard correspondante,
c) en déduire si le sujet présente une activation plaquettaire liée à l'inflammation,
d) si le sujet présente une activation plaquettaire liée à l'inflammation, administrer audit sujet un traitement antiplaquettaire.

Le sujet est notamment tel que défini ci-dessus dans la section « Sujet ».

La maladie inflammatoire est par exemple telle que définie ci-dessus dans la section « maladie inflammatoire ».

L'échantillon biologique est un échantillon provenant d'un sujet, notamment tel que défini ci-dessus dans la section « Echantillon ».

L'échantillon biologique est de préférence un échantillon de plasma riche en plaquettes.

Les étapes a) à c) sont telles que définies ci-dessus dans la section « méthode de diagnostic d'une activation plaquettaire liée à l'inflammation ».

Le traitement antiplaquettaire peut être un traitement diminuant ou inhibant l'activité des plaquettes et/ou un traitement diminuant ou inhibant au moins un des biomarqueurs activé.

### Méthode de suivi de l'efficacité d'un traitement curatif ou préventif d'une maladie inflammatoire

La présente invention a également pour objet une méthode de suivi de l'efficacité d'un traitement curatif ou préventif d'une maladie inflammatoire chez un sujet, comprenant :
a) mesurer la quantité, concentration et/ou proportion des biomarqueurs AKT, PKC, CD62P, CD63, RANTES, TSLP et CD40 ligand dans un échantillon biologique comprenant des plaquettes dudit sujet à un instant t au cours dudit traitement,
b) comparer les résultats obtenus à l'étape a) avec une valeur contrôle standard correspondant et/ou avec une valeur correspondant obtenue avant le début dudit traitement ou à un instant au cours du traitement qui est antérieur à l'instant t, et
c) en déduire si le traitement est efficace,
d) optionnellement, répéter les étapes a) à c).

### La maladie inflammatoire est par exemple telle que définie ci-dessus dans la section « maladie inflammatoire ».

L'échantillon biologique est un échantillon provenant d'un sujet, notamment tel que défini ci-dessus dans la section « Echantillon ».

L'échantillon biologique est de préférence un échantillon de plasma riche en plaquettes.

Le traitement curatif ou préventif peut comprendre un traitement curatif ou préventif de la maladie inflammatoire et/ou un traitement curatif ou préventif d'une activation des plaquettes liées à l'inflammation.

Le traitement curatif ou préventif d'une activation des plaquettes liées à l'inflammation est par exemple un traitement antiplaquettaire tel que défini ci-dessus à la section « Méthode de prévention et/ou traitement d'une maladie inflammatoire associée à une activation plaquettaire liée à l'inflammation ».

Le traitement curatif ou préventif de la maladie inflammatoire peut par exemple comprendre l'administration de corticoïdes, immunomodulateurs, 5-aminosalicylés, broncho-dilatateurs et/ou anti-inflammatoires non stéroïdiens.

Les caractéristiques des étapes a) et b) sont de manière générale telles que définies pour les étapes a) et b) définies dans la section « méthode de détection d'une inflammation plaquettaire ».

Dans la méthode de suivi de l'efficacité d'un traitement définie ci-dessus, l'étape a) comprend de mesurer la quantité, concentration et/ou proportion des biomarqueurs AKT, PKC, CD62P, CD63, RANTES, TSLP et CD40 ligand dans un échantillon biologique comprenant des plaquettes dudit sujet à un instant t au cours du traitement.

En d'autres termes, la mesure est effectuée dans un échantillon biologique comprenant des plaquettes provenant d'un prélèvement effectué à l'instant t.

Dans l'étape b), les résultats obtenus à l'étape a) sont comparés avec une valeur contrôle standard correspondante et/ou avec une valeur correspondante obtenue avant le début dudit traitement ou à un instant au cours du traitement qui antérieur à l'instant t.

Par « valeur correspondante obtenue avant le début dudit traitement », on entend la quantité, concentration et/ou proportion du biomarqueur correspondant mesurée dans un échantillon biologique dudit patient, ledit échantillon ayant été prélevé avant le début du traitement.

Par « valeur correspondante obtenue à un instant au cours du traitement qui antérieur à l'instant t », on entend la quantité, concentration et/ou proportion du biomarqueur correspondant mesurée dans un échantillon biologique dudit patient, ledit échantillon ayant été prélevé après le début du traitement, mais avant l'instant t.

Dans l'étape c), il en est déduit si le traitement est efficace.

Le traitement est par exemple efficace si l'activation plaquettaire liée à l'inflammation diminue ou disparaît.

Par exemple, le traitement est efficace si, pour au moins un biomarqueur activé avant le début dudit traitement ou à un instant au cours du traitement qui antérieur à l'instant t, sa quantité, concentration et/ou proportion mesurée à l'instant t est inférieure à une valeur correspondante obtenue avant le début dudit traitement ou à un instant au cours du traitement qui antérieur à l'instant t.

Par exemple, le traitement est efficace si au moins un biomarqueur n'est plus activé à l'instant t, alors qu'il était activé avant le début dudit traitement et/ou ou à un instant au cours du traitement qui est antérieur à l'instant t.

Par exemple, le traitement n'est pas efficace si, pour au moins un biomarqueur activé avant le début dudit traitement ou à un instant au cours du traitement qui antérieur à l'instant t, sa quantité, concentration et/ou proportion mesurée à l'instant t est supérieure à une valeur correspondante obtenue avant le début dudit traitement ou à un instant au cours du traitement qui antérieur à l'instant t.

Par exemple, le traitement n'est pas efficace si au moins un biomarqueur est activé à l'instant t, alors qu'il n'était pas activé avant le début dudit traitement et/ou ou à un instant au cours du traitement qui est antérieur à l'instant t.

Si le traitement n'est pas efficace, la méthode peut comprendre l'administration d'un nouveau traitement ou d'un traitement complémentaire.

La méthode peut comprendre une étape d) dans laquelle sont répétées les étapes a) à c), en particulier que le traitement soit efficace ou non.

Les étapes a) à c) peuvent être répétées une fois, deux fois, trois fois ou au moins trois fois.

Les étapes a) à c) peuvent par exemple être répétées jusqu'à la guérison du sujet ou la stabilisation de la maladie inflammatoire.

Les étapes a) et c) peuvent être répétées à intervalle de temps régulier ou non.

La période entre deux répétitions peut par exemple être d'au moins une semaine, au moins deux semaines, au moins trois semaines, au moins quatre semaines, au moins deux mois, au moins trois mois, au moins quatre mois, au moins cinq mois, six mois ou au moins six mois, par exemple un an.

La méthode de suivi de l'efficacité d'un traitement curatif ou préventif peut est mise en oeuvre à chaque initiation d'un nouveau traitement ou d'un traitement complémentaire.

### Méthode de suivi de l'évolution d'une maladie inflammatoire associée à une activation plaquettaire liée à l'inflammation

La présente invention a également pour objet une méthode de suivi de l'évolution d'une maladie inflammatoire associée à une activation plaquettaire liée à l'inflammation chez un sujet, comprenant :
a) mesurer la quantité, concentration et/ou proportion des biomarqueurs AKT, PKC, CD62P, CD63, RANTES, TSLP et CD40 ligand dans un échantillon biologique comprenant des plaquettes dudit sujet à deux instants t1 et t2 séparés dans le temps,
b) comparer les résultats obtenus à l'instant t1 et à l'instant t2, à l'étape a), et
c) en déduire si la maladie inflammatoire évolue favorablement,
d) optionnellement, répéter les étapes a) à c).

La maladie inflammatoire est par exemple telle que définie ci-dessus dans la section « maladie inflammatoire ».

L'échantillon biologique est un échantillon provenant d'un sujet, notamment tel que défini ci-dessus dans la section « Echantillon ».

L'échantillon biologique est de préférence un échantillon de plasma riche en plaquettes.

Les caractéristiques des étapes a) et b) sont de manière générale telles que définies pour les étapes a) et b) définies dans la section « méthode de détection d'une inflammation plaquettaire ».

Dans la méthode de suivi de l'évolution d'une maladie inflammatoire définie ci-dessus, l'étape a) comprend de mesurer la quantité, concentration et/ou proportion des biomarqueurs AKT, PKC, CD62P, CD63, RANTES, TSLP et CD40 ligand dans un échantillon biologique comprenant des plaquettes dudit sujet à deux instants t1 et t2 séparés dans le temps.

En d'autres termes, la mesure est effectuée dans un échantillon biologique comprenant des plaquettes provenant d'un prélèvement effectué à l'instant t1, ainsi que dans un échantillon biologique comprenant des plaquettes provenant d'un prélèvement effectué à l'instant t2, l'instant t1 étant antérieur à l'instant t2.

L'intervalle de temps entre t1 et t2 est par exemple d'au moins une semaine, au moins deux semaines, au moins trois semaines, au moins quatre semaines, au moins deux mois, au moins trois mois, au moins quatre mois, au moins cinq mois, six mois ou au moins six mois, par exemple un an.

Dans l'étape b), les résultats obtenus à l'étape a), à l'instant t1 et à l'instant t2 sont comparés entre eux.

Dans l'étape c), il en est déduit si la maladie inflammatoire évolue favorablement.

La maladie inflammatoire évolue favorablement, par exemple si l'activation plaquettaire liée à l'inflammation diminue ou disparaît entre l'instant t1 et t2.

Par exemple, la maladie inflammatoire évolue favorablement si, pour au moins un biomarqueur activé à l'instant t1, sa quantité, concentration et/ou proportion diminue entre l'instant t1 et t2 et/ou si au moins un biomarqueur activé à l'instant t1 n'est plus activé à l'instant t2.

Par exemple, la maladie inflammatoire n'évolue pas favorablement si, pour au moins un biomarqueur activé à l'instant t1, sa quantité, concentration et/ou proportion augmente entre l'instant t1 et t2 et/ou si au moins un biomarqueur est activé à l'instant t2, alors qu'il n'était pas activé à l'instant t1.

La méthode peut comprendre un étape d) dans laquelle sont répétées les étapes a) à c), en particulier que la maladie inflammatoire évolue favorablement ou non.

Les étapes a) à c) peuvent être répétées une fois, deux fois, trois fois ou au moins trois fois.

Les étapes a) à c) peuvent par exemple être répétées jusqu'à la guérison du sujet ou la stabilisation de la maladie inflammatoire.

Les étapes a) et c) peuvent être répétées à intervalle de temps régulier ou non.

La période entre deux répétitions peut par exemple être d'au moins une semaine, au moins deux semaines, au moins trois semaines, au moins quatre semaines, au moins deux mois, au moins trois mois, au moins quatre mois, au moins cinq mois, six mois ou au moins six mois, par exemple un an.

### Méthode de stratification d'un sujet souffrant ou à risque de souffrir d'une maladie inflammatoire

La présente invention a également pour objet une méthode de stratification d'un sujet souffrant ou à risque de souffrir d'une maladie inflammatoire dans une catégorie de sujets avec activation plaquettaire liée à l'inflammation ou dans une catégorie de sujets sans activation plaquettaire liée à l'inflammation, comprenant :
a) mesurer la quantité, concentration et/ou proportion des biomarqueurs AKT, PKC, CD62P, CD63, RANTES, TSLP et CD40 ligand dans un échantillon biologique comprenant des plaquettes dudit sujet,
b) comparer les résultats obtenus à l'étape a) avec une valeur contrôle standard correspondante, et
c) en déduire si le sujet est dans la catégorie de sujets avec une activation plaquettaire liée à l'inflammation ou dans la catégorie de sujets sans activation plaquettaire liée à l'inflammation.

L'échantillon biologique est un échantillon provenant d'un sujet, notamment tel que défini ci-dessus dans la section « Echantillon ».

La maladie inflammatoire est notamment telle que définie ci-dessus dans la section « maladie inflammatoire ».

Les étapes a) et b) sont notamment telles définies ci-dessus dans la section « méthode de détection d'une inflammation plaquettaire ».

En particulier, si la quantité, concentration et/ou proportion d'au moins trois desdits biomarqueurs est supérieure à une valeur contrôle standard NA correspondante (en particulier à la valeur seuil maximale ou à une valeur fixée), il en est déduit que le sujet est dans la catégorie de sujets avec une activation plaquettaire liée à l'inflammation.

En particulier, si la quantité, concentration et/ou proportion d'au moins cinq desdits biomarqueurs est inférieure ou égale à la valeur contrôle standard NA correspondante (en particulier à la valeur seuil maximale ou à une valeur fixée), il en est déduit que le sujet est dans la catégorie de sujets sans activation plaquettaire liée à l'inflammation.

En particulier, si la quantité, concentration et/ou proportion d'au moins trois desdits biomarqueurs est supérieure ou égale à une valeur contrôle standard A correspondante (en particulier à la valeur seuil minimale ou à une valeur fixée), il en est déduit que le sujet est dans la catégorie de sujets avec une activation plaquettaire liée à l'inflammation.

En particulier, si la quantité, concentration et/ou proportion d'au moins cinq desdits biomarqueurs est inférieure à la valeur contrôle standard A correspondante (en particulier à la valeur seuil minimale ou à une valeur fixée), il en est déduit que le sujet est dans la catégorie de sujets sans activation plaquettaire liée à l'inflammation.

### Kit approprié pour la détection d'une activation plaquettaire liée à l'inflammation et son utilisation

La présente description décrit également un kit approprié pour la détection d'une activation plaquettaire liée à l'inflammation comprenant des moyens de détection d'au moins sept biomarqueurs, caractérisé en ce que lesdits au moins sept biomarqueurs sont AKT, PKC, CD62P, CD63, RANTES, TSLP et CD40 ligand.

Les moyens de détection sont par exemple des ligands de détection spécifiques desdits biomarqueurs, optionnellement marqués.

Les ligands de détection spécifiques desdits biomarqueurs sont de préférence des anticorps.

Le kit peut comprendre des moyens permettant de lyser des plaquettes, par exemple un tampon de lyse.

La présente invention a également pour objet l'utilisation d'un kit tel que défini ci-dessus pour la détection d'une activation plaquettaire liée à l'inflammation, en particulier par la mise en oeuvre d'une méthode de détection d'une activation plaquettaire liée à l'inflammation telle que définie ci-dessous.

### Utilisation d'un panel de biomarqueurs

La présente invention a également pour objet l'utilisation, notamment *in vitro,* d'un panel de biomarqueurs comprenant AKT, PKC, CD62P, CD63, RANTES, TSLP et CD40 ligand pour la détection d'une activation plaquettaire liée à l'inflammation, par exemple dans un échantillon biologique.

L'échantillon biologique est notamment tel que défini ci-dessus dans la section « échantillon ».

La présente invention a particulièrement pour objet l'utilisation telle que définie ci-dessus dans laquelle est mesurée la quantité, concentration et/ou proportion des biomarqueurs AKT, PKC, CD62P, CD63, RANTES, TSLP et CD40 ligand.

Mesurer la quantité, concentration et/ou proportion des biomarqueurs AKT, PKC, CD62P, CD63, RANTES, TSLP et CD40 ligand est notamment effectué comme décrit ci-dessus dans la section « méthode de détection d'une inflammation plaquettaire ».

La présente invention a particulièrement pour objet l'utilisation telle que définie ci-dessus dans laquelle est déterminé si au moins trois des biomarqueurs AKT, PKC, CD62P, CD63, RANTES, TSLP et CD40 ligand sont activés ou si au moins cinq des biomarqueurs AKT, PKC, CD62P, CD63, RANTES, TSLP et CD40 ligand sont non activés.

Déterminer si un biomarqueur est activé est notamment effectué comme décrit ci-dessus dans la section « méthode de détection d'une inflammation plaquettaire ».

Par exemple, si au moins trois des biomarqueurs AKT, PKC, CD62P, CD63, RANTES, TSLP et CD40 ligand sont activés, une activation plaquettaire liée à l'inflammation est détectée.

Par exemple, si au moins cinq des biomarqueurs AKT, PKC, CD62P, CD63, RANTES, TSLP et CD40 ligand sont non activés, aucune activation plaquettaire liée à l'inflammation n'est détectée.

D'autres caractéristiques et avantages de l'invention ressortiront mieux des exemples qui suivent, donnés à titre illustratif et non limitatif.

### BREVE DESCRIPTION DU LISTAGE DE SEQUENCE

La séquence SEQ ID NO: 1 correspond au peptide TRAP de séquence SFLLRN.

La séquence SEQ ID NO: 2 correspondant à un agoniste de PAR-4 de séquence AYPGKF.

### FIGURES

Les figures 1 à 8 montrent les variables par ordre d'importance décroissante (telle qu'estimée par un critère de diminution moyenne de précision) dans chaque modèle, avec la variable en ordonnée et l'importance en abscisse.
La Figure 1 présente les variables par ordre d'importance décroissante dans la classe globale.
La Figure 2 présente les variables par ordre d'importance décroissante dans la classe globale correspondant à l'absence de stimulation.
La Figure 3 présente les variables par ordre d'importance décroissante dans la classe correspondant à la stimulation par ADP.
La Figure 4 présente les variables par ordre d'importance décroissante dans la classe correspondant à la stimulation par le collagène.
La Figure 5 présente les variables par ordre d'importance décroissante dans la classe correspondant à la stimulation par le fibrinogène.
La Figure 6 présente les variables par ordre d'importance décroissante dans la classe correspondant à la stimulation par l'agoniste de PAR-1 .
La Figure 7 présente les variables par ordre d'importance décroissante dans la classe correspondant à la stimulation par l'agoniste de PAR-4.
La Figure 8 présente les variables par ordre d'importance décroissante dans la classe correspondant à la stimulation par CD40 soluble.

### EXEMPLES

### Matériel et Méthode

### (i) Plasma riche en plaquette

Des échantillons de sang périphérique de sujets sains (n=10) ont été prélevés et placés dans des tubes sans endotoxine contenant 3,2% de citrate de sodium (Vacutainer^{®}, becton Dickinson, San Jose, CA). Le consentement éclairé des donneurs de sang a été obtenu avant le prélèvement de sang dans un établissement régional du sang, en accord avec la législation française. Les échantillons de sang ont été centrifugés à 192 g pendant 10 minutes à température ambiante pour obtenir un plasma riche en plaquettes.

### (ii) Stimulation des plaquettes

Les tests de réponse des plaquettes ont été effectuées en utilisant un agoniste de PAR-1 (TRAP ou « Thrombin Receptor Activating Peptide » de séquence SEQ ID NO: 1 SFLLRN, 6 µM) (Sigma-Aldrich, Saint Quentin-Fallavier, France), un agoniste de PAR-4 (AYPGKF, 200 µM) (Sigma-Aldrich, Saint Quentin-Fallavier, France), ADP (10 µM), collagène (50 µg/ml), sCD40L (50 ng/ml) ou le fibrinogène (50 µg/ml).

A cet effet, les plaquettes de 10 donneurs ont été soumises à chacune des sept conditions (non stimulées, agoniste de PAR-1, agoniste de PAR-4, ADP, collagène ou sCD40L) et la réponse de 47 paramètres biologiques a été mesurée, à savoir :
- l'activation de biomarqueurs de la membrane : CD62P-%, CD62P-MFI, CD63-%, CD63-MFI,
- l'activation de biomarqueurs solubles : BCA-1, 6Ckine, TSLP, IL-33, GRO alpha, IFNgamma, MDC, CD40ligand, CXCL9/MIG, CCL19/MIP-3b, CCL20/MIP-3a, sérotonien, RANTES, CD62 soluble, et
- l'activation de biomarqueurs intracellulaires : IKBa, IKKa, NFKB1, PKC, AKT, SYK(pY629/30), AXL(pY859), Lyn total, Lyn Y937, PI3K1 10, SYK Py323, SYK total, ERK1/2 pY204/187, Gab1pY285/307/317, Gab2 pY614, PLCg1 pY1253, PLCg2 pY1197/1217, Shc pY349/350, STAT3 pY705, Cbl pY700/731/774, WASH pY291, TEC pY206, RaP GEF1 GRF2 pY504, JAK3 pY785, Gab2 pY584, Gab2pY266, PTEN total, et AXL(pY772).

### (iii) Quantification des biomarqueurs membranaires

Des suspensions de plaquettes sont été mises en incubation avec un anticorps monoclonal approprié, reconnaissant des protéines membranaires exprimées à la surface des plaquettes, dans pendant 30 minutes à température ambiante dans l'obscurité, puis lavées une fois avec un tampon salin phosphate (X1).

Comme toutes les plaquettes expriment de manière constitutive CD41a, ce marqueur (reconnu par un anticorps spécifique conjugué à un fluorochrome - comme par exemple la fluorescéine isothiocyanate) a été utilisé pour définir la fenêtre correspondant aux plaquettes dans les analyses en cytométrie en flux.

Les plaquettes activées sont caractérisées par l'expression de CD62P et CD63, parmi d'autres marqueurs. Des anticorps monoclonaux (ou polyclonaux) conjugués à un fluorochrome - comme par exemple l'allophycocyanine ou à la phycoérythrine - dirigés contre CD62P et CD63 humains ont alors été utilisés pour définir les fenêtres de la population cellulaire à caractériser en fonction de leur activation.

Les analyses en cytométrie en flux ont été réalisées au moyen d'un appareil FACSVantage SE équipé du logiciel CeliQuestS-Pro (BD Biosciences, Le Pont de Claix, France).

Les résultats ont été données en MFI (*Mean Flurescence Intensity*) ou en pourcentage (%). L'immunomarquage CD62P et CD63 permet de préciser le pourcentage de cellules activées parmi les cellules comprises dans la fenêtre d'analyse.

### (iv) Quantification des biomarqueurs solubles

Pour chaque condition, la teneur en protéines des granules α des surnageants plaquettaires a été quantifiée en utilisant la technologie Luminex^{®} comprenant de billes magnétiques spécifiques de différentes cytokines et chimiokines humaines (test de référence HCYTOMAG-60K, HCYP2MAG-62K et HCYP3MAG-63K, Millipore, Molsheim, France). Une molécule du granule δ canonique, à savoir la sérotonine, a été quantifiée par test ELISA (*Enzyme-Linked Immunosorbent Assay*) (IBL International, Hamburg, Allemagne). L'absorbance à 450 nm (ou 405 nm pour la sérotonine) a été mesurée en utilisant un lecteur de plaque ELISA (Magellan Software, Sunrise TM, Tecan Group Ltd., Lyon, France). Les résultats ont été normalisés au pg / 10⁹ plaquettes / ml.

### (v) Quantifications des biomaraueurs intracellulaires

Pour chaque condition, les protéines intracellulaires des plaquettes ont été extraites en utilisant le kit de préparation d'échantillon MILLIPLEX MAP EpiQuant à raison de 3 x 10⁷ plaquettes / ml de tampon de lyse, selon les instructions du fabricant. La quantification des protéines phosphorylées a été effectuée en utilisant la technologie MILLIPLEX MAP EpiQuant avec les cinq panels suivants : MPEQMAG-100K, 102K, 103K, 104K et 110K (Millipore). Les résultats ont été exprimés en pM par 3 x 10⁷ plaquettes / ml.

### Résultats

### (i) Identification d'un panel de biomarqueurs permettant de détecter une activation plaquettaire liée à l'inflammation

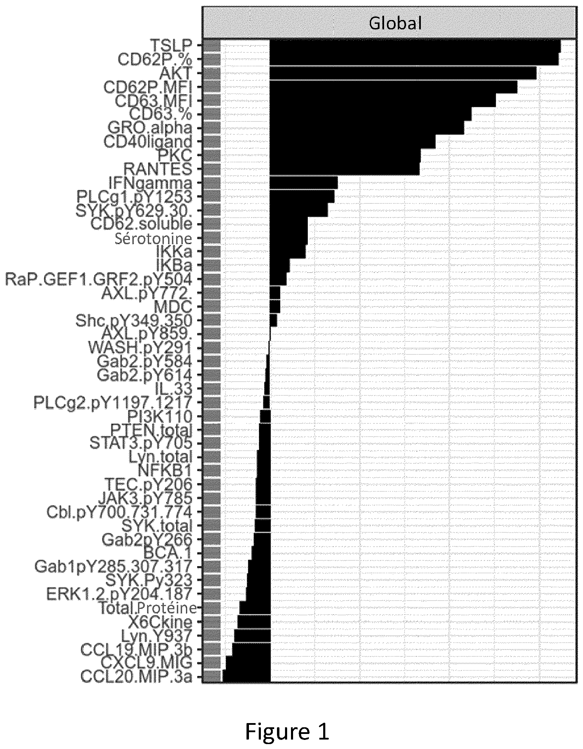

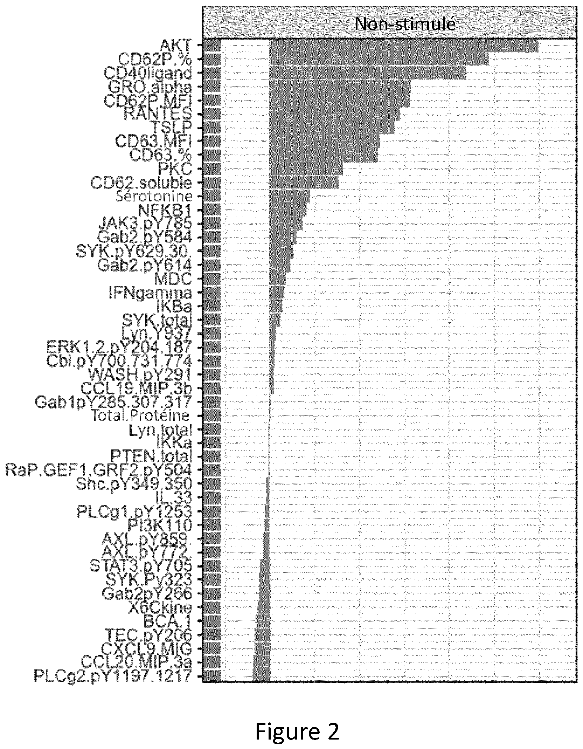

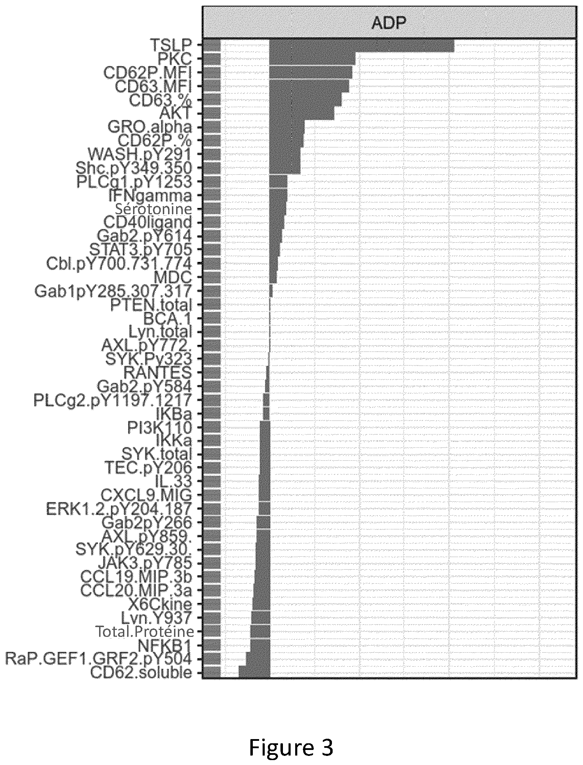

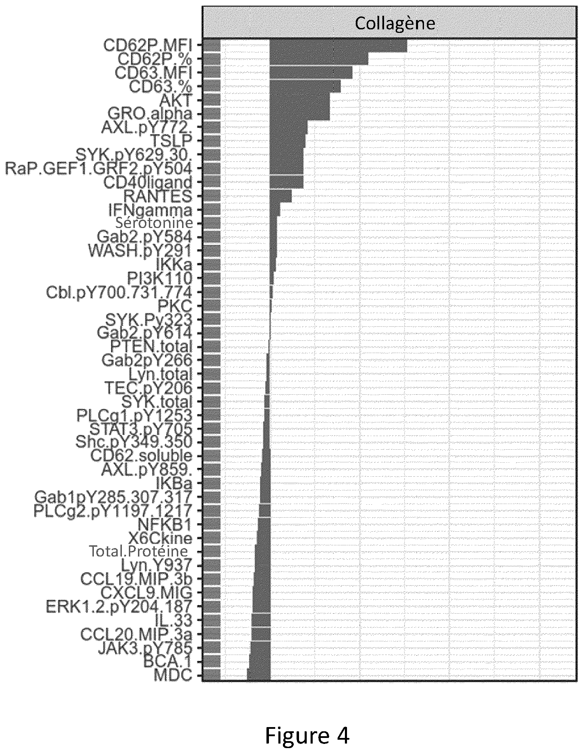

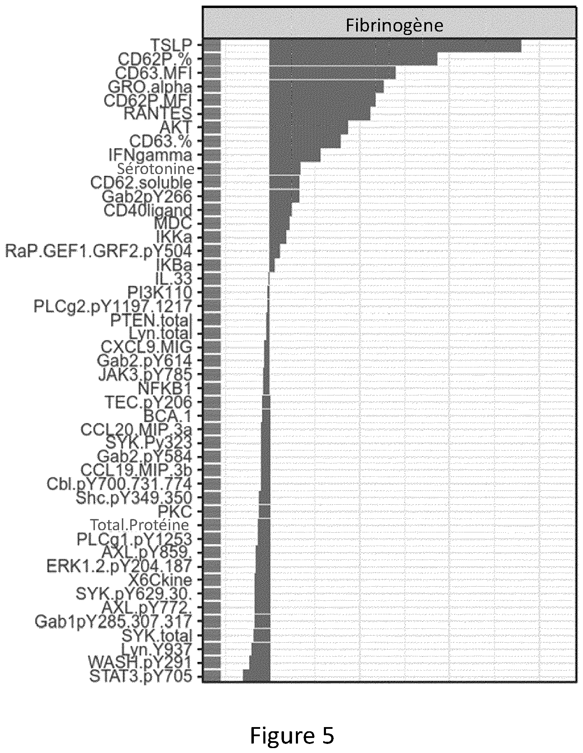

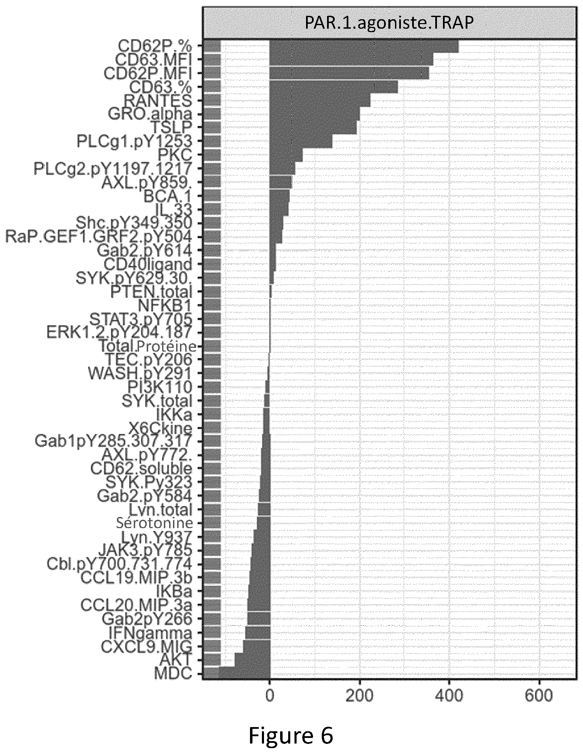

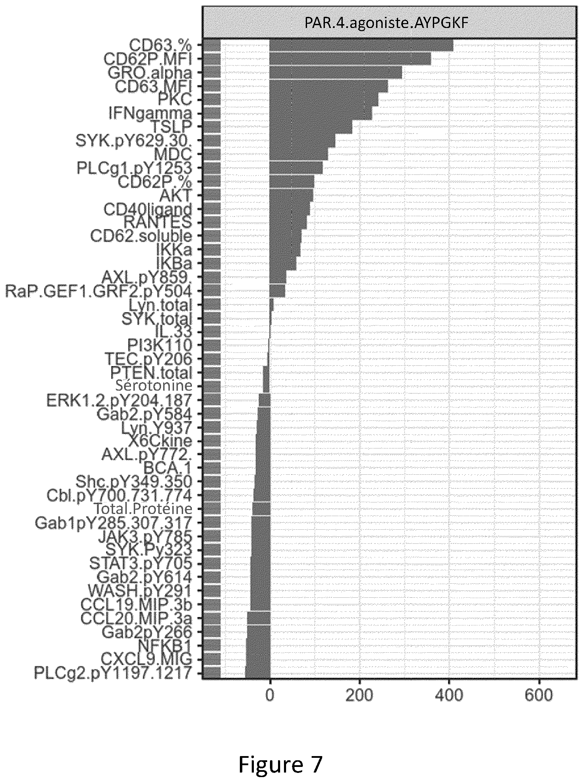

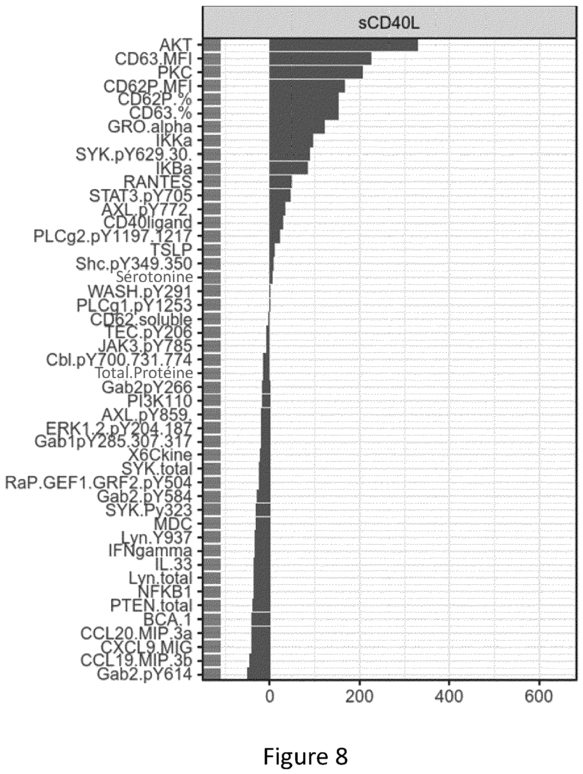

Les figures 1 à 8 montrent les variables par ordre d'importance décroissante (telle qu'estimée par un critère de diminution moyenne de précision) pour le modèle multi classe global (*cf.* *figure 1*) et pour chaque classe individuelle (*cf.* *figures 2 à 8*)*.*

Des validations croisées répétées ont été effectuées pour chacun des 1023 ensembles de variables, à partir des 10 variables les plus importantes identifiées à la figure 1. Le résultat est présenté dans le tableau 1 ci-dessous.

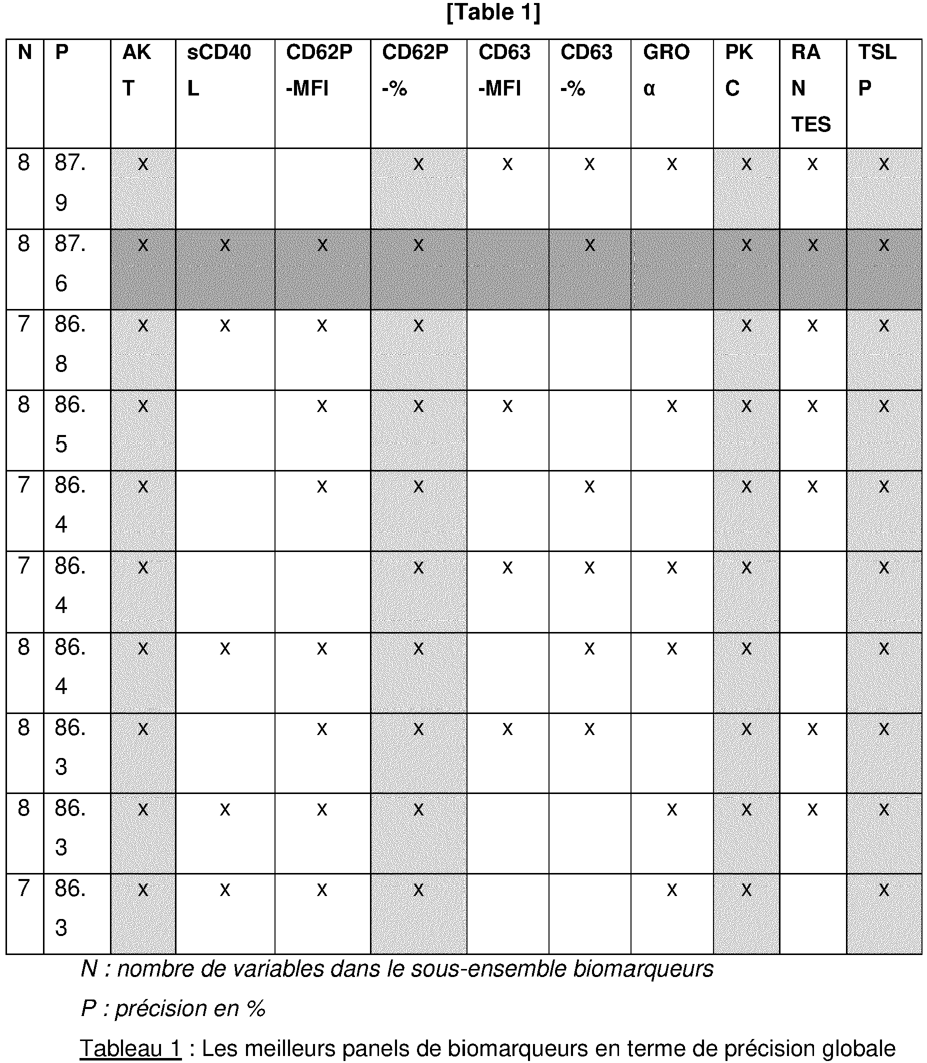

A partir des résultats présentés dans le tableau 1 a été défini un panel de biomarqueurs comprenant AKT, PKC, CD62P, CD63, RANTES, TSLP et CD40 ligand qui permet de déterminer avec une très haute précision la présence d'une activation plaquettaire liée à l'inflammation.

### (ii) Identification de biomarqueurs spécifiques de l'activation d'une voie de signalisation intra-plaauettaire

Cette analyse biomathématique a également permis d'identifier des biomarqueurs spécifiques de l'activation d'une voie de signalisation intra-plaquettaire, telles que:
- NFKB1 et Shc pY349/350, associés à une stimulation par l'agoniste de PAR-1 ;
- SYK (pY629/30), associé à une stimulation par l'agoniste de PAR-4;
- BCA-1, CD63-%, Gab2 pY614, IFNy et MDC, associés à une stimulation par l'agoniste de PAR-1 et l'agoniste de PAR-4;
- PKC, associé à une stimulation par l'agoniste de PAR-4 et l'ADP ;
- CD63-MFI, associé à une stimulation par l'agoniste de PAR-1, l'agoniste de PAR-4 et le collagène ;
- CD62 soluble et CD62P-MFI, associés à une stimulation par l'agoniste de PAR-1, l'agoniste de PAR-4, le collagène et l'ADP;
- CD62P-%, GROα et RANTES, associés à une stimulation par l'agoniste de PAR-1, l'agoniste de PAR-4, le collagène, l'ADP et sCD40L;
- TSLP, associés à une stimulation par le fibrinogène, l'agoniste de PAR-4, le collagène, sCD40L,
- AKT, associés à une stimulation par sCD40L, l'agoniste de PAR-1, l'agoniste de PAR-4, le fibrinogène, le collagène et l'ADP, et
- CDL40, associé à une stimulation par l'agoniste de PAR-1, l'agoniste de PAR-4 -, le fibrinogène, le collagène et l'ADP. (*cf. tableau* 2 ci-dessous).

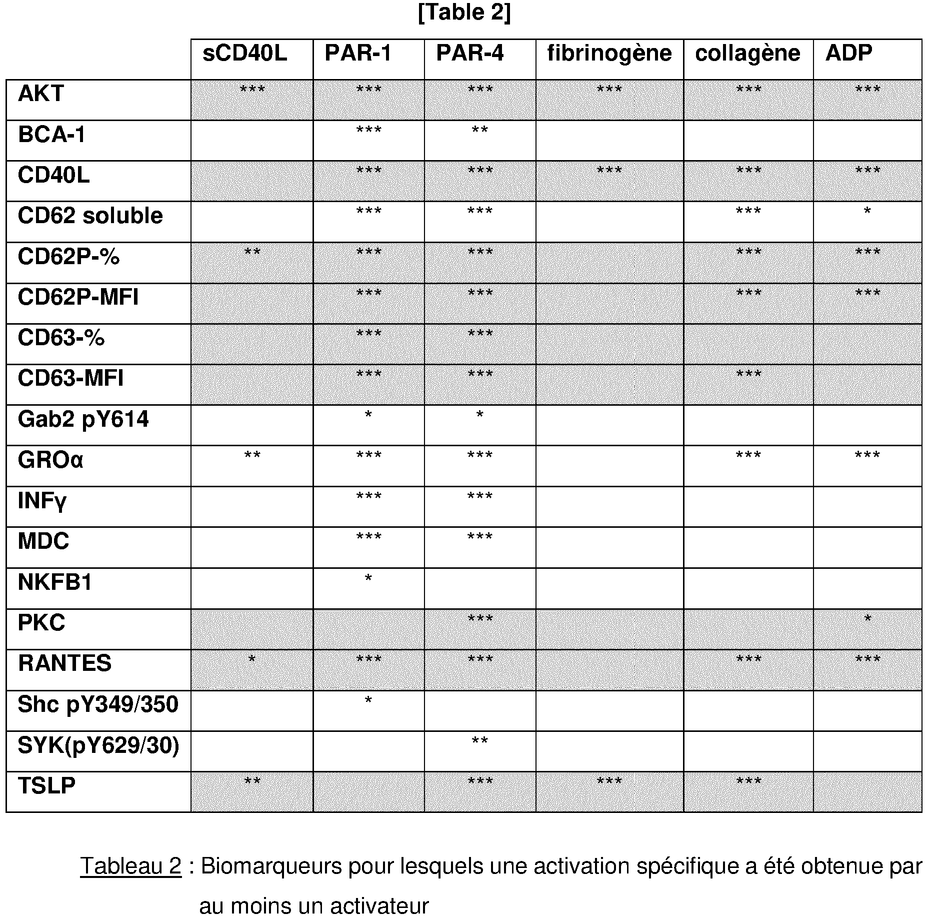

## Revendications

1. Méthode de détection d'une activation plaquettaire liée à l'inflammation, comprenant de mesurer la quantité, concentration et/ou proportion des biomarqueurs AKT, PKC, CD62P, CD63, RANTES, TSLP et CD40 ligand dans un échantillon biologique comprenant des plaquettes.

2. Méthode de diagnostic d'une activation plaquettaire liée à l'inflammation chez un sujet, comprenant :
a) mesurer la quantité, concentration et/ou proportion des biomarqueurs AKT, PKC, CD62P, CD63, RANTES, TSLP et CD40 ligand dans un échantillon biologique comprenant des plaquettes dudit sujet,
b) comparer les résultats obtenus à l'étape a) avec une valeur contrôle standard correspondante, et
c) en déduire si le sujet présente une activation plaquettaire liée à l'inflammation.

3. Méthode selon la revendication 2, **caractérisée en ce que** l'étape c) comprend d'en déduire si une voie de signalisation intra-plaquettaire spécifique est activée.

4. Méthode de suivi de l'efficacité d'un traitement curatif ou préventif d'une maladie inflammatoire chez un sujet, comprenant :
a) mesurer la quantité, concentration et/ou proportion des biomarqueurs AKT, PKC, CD62P, CD63, RANTES, TSLP et CD40 ligand dans un échantillon biologique comprenant des plaquettes dudit sujet à un instant t au cours dudit traitement,
b) comparer les résultats obtenus à l'étape a) avec une valeur contrôle standard correspondante et/ou avec une valeur correspondante obtenue avant le début dudit traitement ou à un instant au cours du traitement qui est antérieur à l'instant t,
c) en déduire si le traitement est efficace, et
d) optionnellement, répéter les étapes a) à c).

5. Méthode de suivi de l'évolution d'une maladie inflammatoire associée à une activation plaquettaire liée à l'inflammation chez un sujet, comprenant :
a) mesurer la quantité, concentration et/ou proportion des biomarqueurs AKT, PKC, CD62P, CD63, RANTES, TSLP et CD40 ligand dans un échantillon biologique comprenant des plaquettes dudit sujet à deux instants t1 et t2 séparés dans le temps,
b) comparer les résultats à l'instant t1 et à l'instant t2 obtenus à l'étape a),
c) en déduire si la maladie inflammatoire évolue favorablement, et
d) optionnellement, répéter les étapes a) à c).

6. Méthode de stratification d'un sujet souffrant ou à risque de souffrir d'une maladie inflammatoire dans une catégorie de sujets avec activation plaquettaire liée à l'inflammation ou dans une catégorie de sujets sans activation plaquettaire liée à l'inflammation, comprenant :
a) mesurer la quantité, concentration et/ou proportion des biomarqueurs AKT, PKC, CD62P, CD63, RANTES, TSLP et CD40 ligand dans un échantillon biologique comprenant des plaquettes dudit sujet,
b) comparer les résultats obtenus à l'étape a) avec une valeur contrôle standard correspondante, et
c) en déduire si le sujet est dans la catégorie de sujets avec activation plaquettaire liée à l'inflammation ou dans la catégorie de sujets sans activation plaquettaire liée à l'inflammation.

7. Méthode selon l'une des revendications 4 à 6, **caractérisée en ce que** ladite maladie inflammatoire est sélectionnée dans le groupe consistant en athérosclérose, inflammation pulmonaire, polyarthrite rhumatoïde (PR), maladie inflammatoire chronique de l'intestin (MICI), sepsis, sepsis sévère, choc septique, cancer et leurs combinaisons.

8. Méthode selon l'une des revendications 1 à 7, **caractérisée en ce que** l'échantillon biologique est un échantillon de plasma riche en plaquettes.

9. Utilisation *in vitro* d'un kit comprenant des moyens de détection d'au moins sept biomarqueurs, **caractérisé en ce que** lesdits au moins sept biomarqueurs sont AKT, PKC, CD62P, CD63, RANTES, TSLP et CD40 ligand pour la détection d'une activation plaquettaire liée à l'inflammation.

10. Utilisation *in vitro* d'un panel de biomarqueurs comprenant AKT, PKC, CD62P, CD63, RANTES, TSLP et CD40 ligand pour la détection d'une activation plaquettaire liée à l'inflammation.

## Patentansprüche

1. Verfahren zum Detektieren einer entzündungsbedingten Thrombozytenaktivierung, umfassend ein Messen der Menge, Konzentration und/oder des Anteils der Biomarker AKT, PKC, CD62P, CD63, RANTES, TSLP und CD40-Ligand in einer biologischen Probe, umfassend Thrombozyten.

2. Verfahren zum Diagnostizieren einer entzündungsbedingten Thrombozytenaktivierung bei einem Subjekt, umfassend:
a) Messen der Menge, Konzentration und/oder des Anteils der Biomarker AKT, PKC, CD62P, CD63, RANTES, TSLP und CD40 Ligand in einer biologischen Probe, umfassend Thrombozyten des Subjekts,
b) Vergleichen der in Schritt a) erlangten Resultate mit einem entsprechenden standardmäßigen Kontrollwert, und
c) daraus Ableiten, ob das Subjekt eine entzündungsbedingte Thrombozytenaktivierung aufweist.

3. Verfahren nach Anspruch 2, **dadurch gekennzeichnet, dass** Schritt c) umfasst, daraus abzuleiten, ob ein spezifischer thrombozyteninterner Signalweg aktiviert ist.

4. Verfahren zur Überwachung der Wirksamkeit einer heilenden oder vorbeugenden Behandlung einer entzündlichen Erkrankung bei einem Subjekt, umfassend:
a) Messen der Menge, Konzentration und/oder des Anteils der Biomarker AKT, PKC, CD62P, CD63, RANTES, TSLP und CD40 Ligand in einer biologischen Probe, umfassend Thrombozyten des Subjekts zu einem Zeitpunkt t während der Behandlung,
b) Vergleichen der in Schritt a) erlangten Resultate mit einem entsprechenden standardmäßigen Kontrollwert und/oder mit einem entsprechenden Wert, der vor Beginn der Behandlung oder zu einem Zeitpunkt während der Behandlung, der vor dem Zeitpunkt t ist, erlangt wird,
c) daraus Ableiten, ob die Behandlung wirksam ist, und
d) optional Wiederholen der Schritte a) bis c).

5. Verfahren zur Überwachung des Verlaufs einer entzündlichen Erkrankung, die mit einer entzündungsbedingten Thrombozytenaktivierung bei einem Subjekt assoziiert ist, umfassend:
a) Messen der Menge, Konzentration und/oder des Anteils der Biomarker AKT, PKC, CD62P, CD63, RANTES, TSLP und CD40 Ligand in einer biologischen Probe, umfassend Thrombozyten des Subjekts zu zwei zeitlich getrennten Zeitpunkten t1 und t2,
b) Vergleichen der in Schritt a) erlangten Resultate zum Zeitpunkt t1 und zum Zeitpunkt t2,
c) daraus Ableiten, ob die entzündliche Erkrankung einen günstigen Verlauf nimmt, und
d) optional Wiederholen der Schritte a) bis c).

6. Verfahren zur Stratifizierung einer Person, die an einer entzündlichen Erkrankung leidet oder gefährdet ist, an einer entzündlichen Erkrankung zu leiden, in eine Kategorie von Subjekten mit entzündungsbedingter Thrombozytenaktivierung oder in eine Kategorie von Subjekten ohne entzündungsbedingte Thrombozytenaktivierung, umfassend:
a) Messen der Menge, Konzentration und/oder des Anteils der Biomarker AKT, PKC, CD62P, CD63, RANTES, TSLP und CD40 Ligand in einer biologischen Probe, umfassend Thrombozyten des Subjekts,
b) Vergleichen der in Schritt a) erlangten Resultate mit einem entsprechenden standardmäßigen Kontrollwert, und
c) daraus ableiten, ob das Subjekt in die Kategorie von Subjekten mit entzündungsbedingter Thrombozytenaktivierung oder in die Kategorie von Subjekten ohne entzündungsbedingte Thrombozytenaktivierung fällt.

7. Verfahren nach einem der Ansprüche 4 bis 6, **dadurch gekennzeichnet, dass** die entzündliche Erkrankung ausgewählt ist aus der Gruppe, bestehend aus Atherosklerose, Lungenentzündung, rheumatoide Arthritis (RA), chronisch entzündlicher Darmerkrankung (CED), Sepsis, schwerer Sepsis, septischem Schock, Krebs und Kombinationen davon.

8. Verfahren nach einem der Ansprüche 1 bis 7, **dadurch gekennzeichnet, dass** die biologische Probe eine Probe von thrombozytenreichem Plasma ist.

9. Verwendung *in vitro* eines Kits, umfassend Einrichtungen zum Detektieren von mindestens sieben Biomarkern, **dadurch gekennzeichnet, dass** die mindestens sieben Biomarker AKT, PKC, CD62P, CD63, RANTES, TSLP und CD40-Ligand zum Detektieren einer entzündungsbedingten Thrombozytenaktivierung sind.

10. Verwendung *in vitro* eines Panels von Biomarkern, umfassend AKT, PKC, CD62P, CD63, RANTES, TSLP und CD40-Ligand zum Detektieren einer entzündungsbedingten Thrombozytenaktivierung.

## Claims

1. A method for detecting an inflammation-related platelet activation comprising measuring the quantity, concentration and/or proportion of the biomarkers AKT, PKC, CD62P, CD63, RANTES, TSLP and CD40 ligand in a biological sample comprising platelets.

2. A method of diagnosis for inflammation-related platelet activation in an individual, comprising:
a) measuring the quantity, concentration and/or proportion of the biomarkers AKT, PKC, CD62P, CD63, RANTES, TSLP and CD40 ligand in a biological sample comprising platelets of said individual,
b) comparing the results obtained during the step a) with a corresponding standard control value, and
c) deducing from the above if the individual has inflammation-related platelet activation.

3. The method according to claim 2, **characterized in that** the step c) comprises deducing therefrom whether a specific intra-platelet signaling pathway is activated.

4. A method for monitoring the efficacy of a curative or preventive treatment for an inflammatory disease in an individual, comprising:
a) measuring the quantity, concentration and/or proportion of the biomarkers AKT, PKC, CD62P, CD63, RANTES, TSLP and CD40 ligand in a biological sample comprising platelets of said individual at a time t during said treatment,
b) comparing the results obtained during the step a) with a corresponding standard control value and/or with a corresponding value obtained before the start of said treatment or at a time during the treatment, which is earlier than time t,
c) deducing therefrom whether the treatment is effective, and
d) optionally, repeating the steps a) to c).

5. A method of monitoring the evolution of an inflammatory disease associated with inflammation-related platelet activation in an individual, comprising:
a) measuring the quantity, concentration and/or proportion of the biomarkers AKT, PKC, CD62P, CD63, RANTES, TSLP and CD40 ligand in a biological sample comprising platelets of said individual at two times t1 and t2, separated in time,
b) comparing the results at time t1 and time t2 obtained during the step a),
c) deducing therefrom whether the inflammatory disease evolves favorably, and
d) optionally, repeating the steps a) to c).

6. A stratification method for an individual with or at risk of suffering from inflammatory disease in a class of individuals with inflammation-related platelet activation or in a class of individuals without any inflammation-related platelet activation, comprising:
a) measuring the quantity, concentration and/or proportion of the biomarkers AKT, PKC, CD62P, CD63, RANTES, TSLP and CD40 ligand in a biological sample comprising platelets of said individual,
b) comparing the results obtained during the step a) with a corresponding standard control value, and
c) deducing therefrom whether the individual belongs to the class of individuals with inflammation-related platelet activation or to the class of individuals without inflammation-related platelet activation.

7. The method according to one of claims 4 to 6, **characterized in that** said inflammatory disease is selected from the group consisting of atherosclerosis, pulmonary inflammation, rheumatoid arthritis (RA), chronic inflammatory bowel disease (IBD), sepsis, severe sepsis, septic shock, cancer and combinations thereof.

8. The method according to one of claims 1 to 7, **characterized in that** the biological sample is a platelet-rich plasma sample.

9. *In vitro* use of a kit comprising means for detecting at least seven biomarkers, **characterized in that** said at least seven biomarkers are AKT, PKC, CD62P, CD63, RANTES, TSLP and CD40 ligand for the detection of an inflammation-related platelet activation.

10. *In vitro* use of a biomarker panel including AKT, PKC, CD62P, CD63, RANTES, TSLP and CD40 ligand for the detection of an inflammation-related platelet activation.
